# EUROPEAN PATENT SPECIFICATION

(11) **EP 2 289 412 B1**
(45) Date of publication and mention of the grant of the patent: **30.10.2013**
(21) Application number: 10167968.6
(22) Date of filing: 30.06.2010
(51) Int. Cl.: A61B 5/08, A61B 5/113

(54) **Unconstrained wearable spirometer apparatus, system, and measurement method using the same**
Behinderungsarm tragbare Spirometervorrichtung, System und Messverfahren zu dessen Verwendung
Appareil de spiromètre non contraint portable, système, et procédé de mesure l'utilisant

(30) Priority: 01.09.2009 KR 20090082105
(43) Date of publication of application: 02.03.2011
(73) Proprietor: Electronics and Telecommunications Research Institute, Daejeon 305-700 (KR)
(72) Inventor: Jeong, Ji Wook, Daejeon (KR); Jang, Yong Won, Daejeon (KR); Kim, Seung Hwan, Daejeon (KR); Park, Seon Hee, Daejeon (KR)
(74) Representative: Betten & Resch

(56) References cited:
- WO-A1-2006/066773
- DE-A1- 4 229 073
- US-A1- 2008 300 503
- LORIGA ET AL: "Textile Sensing Interfaces for Cardiopulmonary Signs Monitoring", ENGINEERING IN MEDICINE AND BIOLOGY SOCIETY, 2005. IEEE-EMBS 2005. 27T H ANNUAL INTERNATIONAL CONFERENCE OF THE SHANGHAI, CHINA 01-04 SEPT. 2005, PISCATAWAY, NJ, USA,IEEE LNKD- DOI:10.1109/IEMBS.2005.1616209, 1 January 2005 (2005-01-01), pages 7349-7352, XP031000895, ISBN: 978-0-7803-8741-6

## Description

### BACKGROUND

### 1. Field of the Invention

The present invention relates to a spirometer apparatus and a method of measuring lung capacity using the same, and more particularly, to an unconstrained wearable spirometer apparatus and a measurement method using the same.

### 2. Discussion of Related Art

When a respiration bio-signal such as lung capacity is measured, inhalation and exhalation are directly collected from outside the human body or inhalation and exhalation are filtered to electrically measure a rate of airflow.

However, such methods require external equipment whenever measurement is performed and the holding of an apparatus between the teeth.

Document US 2008/300503 A1 discloses a method and system provided for monitoring a respiratory signal by radio. The method includes the steps of: converting a change in electric resistance, which is caused by a change in abdominal circumference measured through a rubber waistband that is made of conductive rubber and is mounted on a lower garment of a testee during respiration, into a voltage signal, performing A/D conversion on the voltage signal, and transmitting the converted digital signal to a short distance by radio using a wireless communication protocol for ZigBee; and receiving the respiratory signal transmitted by radio, transmitting it to a computer unit by wire through an RS-232 port that is a serial communication port, and enabling a tester to monitor the respiratory signal through a screen.

Document WO 2006/066773 discloses a conductor particularly for the electrical connection to each other of two distant points whose distance can vary over time, comprising a body made of elastic material on which conducting fibers are wound.

### SUMMARY OF THE INVENTION

The present invention is directed to a wearable spirometer apparatus that may be worn as clothing, detect a change in one's chest in real time, and estimate a change in lung volume, so that lung capacity can be measured.

One aspect of the present invention provides a wearable spirometer apparatus according to claim 1.

The apparatus may further include a display module displaying lung capacity information calculated by the adder.

The variable resistor may be in the form of a band or clothing.

The resistance-voltage converter may include a voltage divider circuit to convert the resistance of the variable resistor into a voltage.

The transmitter may externally transmit the lung capacity information using a wired or wireless communication method.

Another aspect of the present invention provides a method of measuring lung capacity using a wearable spirometer apparatus according to claim 7.

The method may further include displaying the lung capacity information after calculating the lung capacity.

The method may further include analyzing and displaying the externally transmitted lung capacity information after the calculated lung capacity information is externally transmitted.

The conductive yarns may be spaced apart at uniform intervals and be in the form of a band or clothing.

The voltage may be converted from the resistance, by a voltage divider circuit.

The lung capacity information may be externally transmitted using a wired or wireless communication method.

Still another aspect of the present invention provides to a wearable spirometer system according to claim 6.

Further advantageous embodiments are defined in dependent claims.

### BRIEF DESCRIPTION OF THE DRAWINGS

The above and other features and advantages of the present invention will become more apparent to those of ordinary skill in the art by describing in detail exemplary embodiments thereof with reference to the attached drawings in which:

FIG. 1A is a block diagram of a wearable spirometer apparatus according to an exemplary embodiment;

FIG. 1B illustrates a person wearing the wearable spirometer apparatus of FIG. 1A;

FIG. 2 is a block diagram of a calculator illustrated in FIG. 1A;

FIG. 3 is a block diagram of a wearable spirometer system according to an exemplary embodiment of the present invention;

FIG. 4 is a flowchart illustrating a method of measuring lung capacity using a wearable spirometer apparatus according to an exemplary embodiment of the present invention; and

FIG. 5 is a graph showing changes in estimated lung capacity over time when a wearer breathes.

### DETAILED DESCRIPTION OF EMBODIMENTS

The present invention will be described more fully hereinafter with reference to the accompanying drawings, in which exemplary embodiments of the invention are shown. This invention may, however, be embodied in different forms and should not be construed as limited to the embodiments set forth herein. In the drawings, portions irrelevant to a description of the present invention are omitted for clarity, and like reference numerals denote like elements..

Throughout the specification, it will be understood that when a portion "includes" an element, it is not intended to exclude other elements but can further include other elements. Also, terms, such as "portion," "system" and "module" may be used herein to refer to units for processing at least one function or operation which are implemented in hardware, software, or a suitable combination of both.

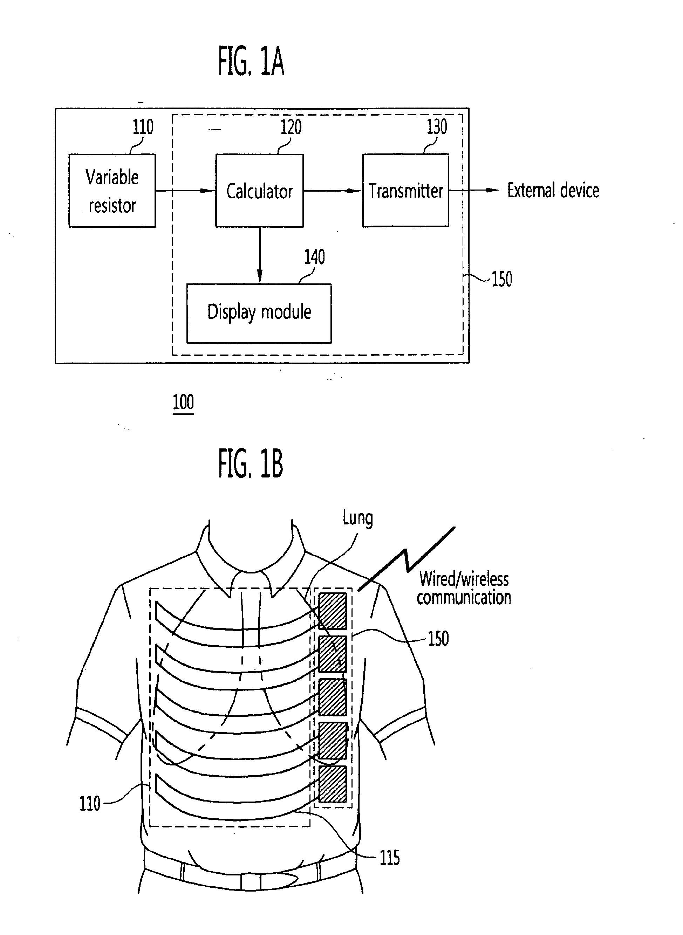

FIG. 1A is a block diagram of a wearable spirometer apparatus according to an exemplary embodiment, and FIG. 1B illustrates a person wearing the wearable spirometer apparatus of FIG. 1A.

Referring to FIG. 1A, a wearable spirometer apparatus 100 according to the present invention includes a variable resistor 110 and a reading module 150, and the reading module 150 includes a calculator 120, a transmitter 130 and a display module 140.

The variable resistor 110 includes a resistive conductive yarn, the length of which varies according to a change in circumference of one's chest in breathing. A resistance is changed according to a change in length of the conductive yarn. The variable resistor 110 includes a plurality of resistive conductive yarns, and the conductive yarns are spaced apart from each other on the chest at uniform intervals.

The conductive yarns include a conductive fiber and an elastic yarn. Because of elasticity of the elastic yarn when the circumference of one's chest is increased while breathing, the conductive yarns may be lengthened.

The conductive fiber includes a carbon fiber or a metal line. The conductive yarn may have a shape in which the conductive fiber is twisted with the elastic yarn. For example, the conductive fibers may be wound around the elastic yarn, or the conductive fibers may be wound twice in the opposite direction around the elastic yarn.

Referring to FIG. 1B, the variable resistor 110 including the plurality of resistive conductive yarns 115 spaced apart at uniform intervals may be formed in the shape of a band wrapping around a body to be independently worn. Alternatively, it may be inserted into clothing.

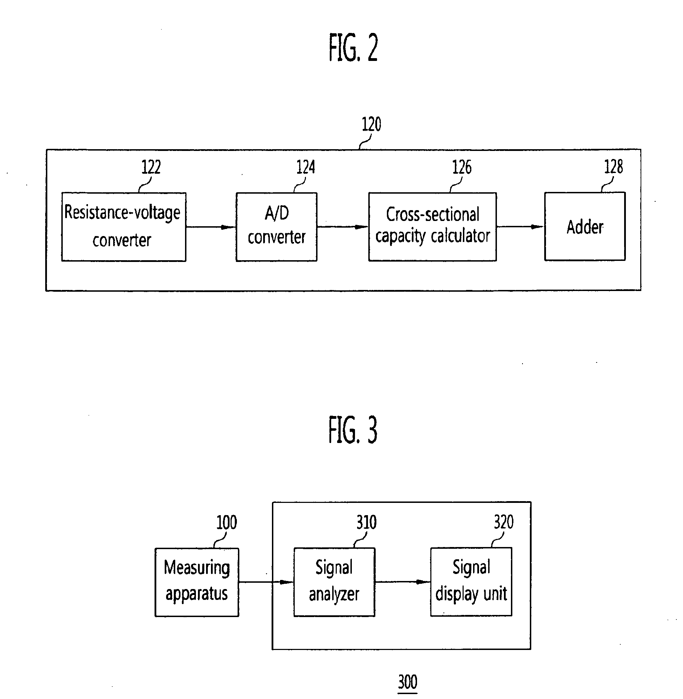

The calculator 120 performs conversion, changes and addition to calculate lung capacity from a resistance of the variable resistor 110. A detailed constitution of the calculator 120 will be described with reference to FIG. 2.

The transmitter 130 transmits lung capacity information calculated by the calculator 120 to an external device. The transmitter 130 may transmit the lung capacity information to the external device using a wired or wireless communication method.

The display module 140 displays the lung capacity information calculated by the calculator 120. It may inform users of results of measuring lung capacities through the display module 140 such as a liquid crystal display, etc.

FIG. 2 is a block diagram illustrating the calculator of FIG. 1A in more detail.

Referring to FIG. 2, the calculator 120 includes a resistance-voltage converter 122, an A/D converter 124, a cross-sectional capacity calculator 126 and an adder 128.

The resistance-voltage converter 122 converts a resistance of the variable resistor 110 into a voltage. For this purpose, the resistance-voltage converter 122 may include one or more voltage divider circuits.

The voltage divider circuit includes a reference resistance and a variable resistance that are serially connected between a power voltage terminal and a ground terminal. A portion formed of a resistive conductive yarn corresponds to the variable resistance. When breathing, the circumference of one's chest changes, causing the length of the resistive conductive yarn and the resistance to change, and the voltage applied to the variable resistance varies. Therefore, when the voltage is measured, the resistance may be converted into a voltage.

The A/D converter 124 converts an analog voltage converted by the resistance-voltage converter 122 into a digital voltage, and expresses the converted results numerically. The A/D converter 124 includes a plurality of A/D converting portions.

The cross-sectional capacity calculator 126 calculates a change in cross-sectional capacity of a chest according to the digital value digitized by the A/D converter 124. More specifically, a change in cross-sectional capacity is calculated by applying a conversion formula. For example, when a user wears a spirometer apparatus according to the present invention and inhales, the circumference of the user's chest increases, and thus the length of the conductive yarn also increases. Assuming that the user's upper body forms a cross-sectional sphere, the radius of the circle made by the user's body increases, and thus the area of the circle increases as well. Applying such a principle, a change in cross-sectional capacity of one's chest may be calculated.

The adder 128 adds the changes in cross-sectional capacity calculated by the cross-sectional capacity calculator 126 and calculates lung capacity. The conductive yarn of the variable resistor 110 may be repeatedly attached to a body within the height from shoulder to navel. Therefore, each change in cross-sectional capacity is added up per height, so that a change in volume of an effective lung capacity according to a breathing exercise may be calculated.

In the exemplary embodiment of the present invention, the variable resistor 110 includes a plurality of resistive conductive yarns, and each resistive conductive yarn may be connected to, e.g., a voltage divider circuit of the resistance-voltage converter 122, and to one of a plurality of A/D converting portions of the A/D converter. That is, a conductive yarn is connected to a voltage divider circuit, the cross-sectional capacity is calculated via one A/D converting portion, and the calculated cross-sectional capacity is repeatedly added with respect to the conductive yarns, so that lung capacity may be obtained.

FIG. 3 is a block diagram of a wearable spirometer system according to an exemplary embodiment of the present invention.

Referring to FIG. 3, the wearable spirometer system of the present invention includes a wearable spirometer apparatus 100, a signal analyzer 310, and a signal display unit 320. The signal analyzer 310 and the signal display unit 320 may be included in an external device 300 such as a personal digital assistant (PDA).

The signal analyzer 310 analyzes lung capacity information transmitted from the wearable spirometer apparatus 100, and the signal display unit 320 displays the lung capacity information analyzed by the signal analyzer 310.

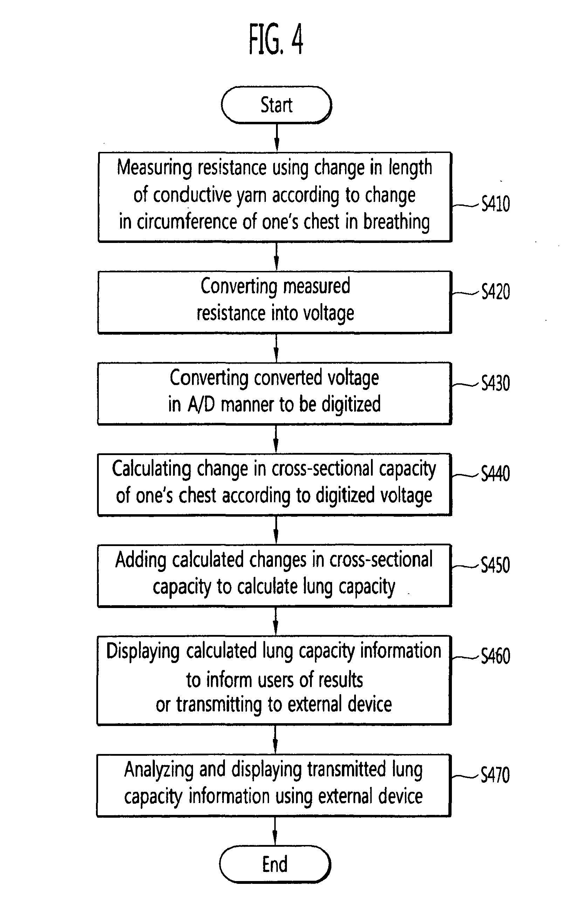

FIG. 4 is a flowchart illustrating a method of measuring lung capacity using a wearable spirometer apparatus according to an exemplary embodiment of the present invention.

Since the detailed exemplary embodiment of a method of measuring lung capacity using the wearable spirometer apparatus according to an exemplary embodiment of the present invention is as described above, the operation processes thereof will be briefly described.

Referring to FIG. 4, a resistance is measured using a change in length of a conductive yarn according to a change in circumference of one's chest in breathing (S410).

Afterwards, the measured resistance is converted into a voltage (S420), and the converted voltage is A/D converted to be digitized (S430).

Next, a change in cross-sectional capacity of one's chest according to the digitized voltage is calculated (S440), and the calculated changes in cross-sectional capacity are added to calculate lung capacity (S450).

Then, the calculated lung capacity information is displayed to inform users of the results or transmitted to an external device (S460).

Next, the transmitted lung capacity information is analyzed and displayed by the external device (S470).

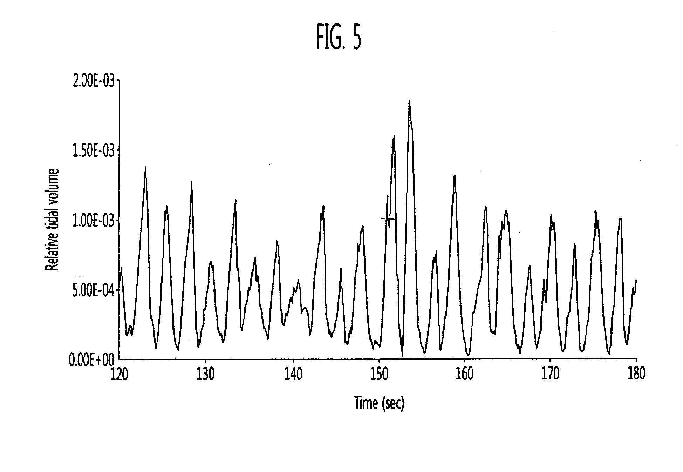

FIG. 5 is a graph showing changes in estimated lung capacity over time when a wearer breathes.

Referring to FIG. 5, it is observed that when a wearer breathes, estimated lung capacity calculated based on a measured voltage changes in the same manner as a respiration cycle.

According to a wearable spirometer apparatus according to the present invention, lung capacity can be measured by wearing such an apparatus in clothing form without the use of an external apparatus or device.

Further, such an apparatus enables serial monitoring, the results of measuring lung capacity to be displayed for users to see, and the results of measuring lung capacity to be transmitted to an external device using a wired/wireless communication method. Therefore, when any abnormal conditions (e.g., sleep apnea) arise during a breathing exercise of a user, such conditions can be noticed by others to prevent future accidents.

In the drawings and specification, there have been disclosed typical exemplary embodiments of the invention and, although specific terms are employed, they are used in a generic and descriptive sense only and not for purposes of limitation. As for the scope of the invention, it is to be set forth in the following claims. Therefore, it will be understood by those of ordinary skill in the art that various changes in form and details may be made therein without departing from the scope of the present invention as defined by the following claims.

## Claims

1. A. wearable spirometer apparatus (100), comprising:
a variable resistor (110) including a plurality of conductive yarns (115), each of which has a length which changes according to a change in circumference of one's chest when breathing, and has a resistance which changes according to a change in length of the conductive yam, wherein said conductive yarns are spaced apart at uniform intervals;
a resistance-voltage converter (122) configured to convert the resistance of each of the conductive yarns of the variable resistor into a voltage;
an analog-digital converter (124) configured to convert each voltage in an analog-digital manner, and digitizing the converted results;
a cross-sectional capacity calculator (126) configured to calculated changes in cross-sectional capacity of one's chest according to the digitized voltages by applying a conversion formula;
an adder (128) configured to add each calculated change in cross-sectional capacity per height, and to calculate lung capacity; and
a transmitter (130) configured to externally transmit the calculated lung capacity information.

2. The apparatus (100) of claim 1, further comprising a display module (140) configured to display the lung capacity information calculated by the adder.

3. The apparatus (100) of any of the preceding claims, wherein the variable resistor (110) is in the form of a band or clothing.

4. The apparatus (100) of any of the preceding claims, wherein the
resistance-voltage converter (122) includes a voltage divider circuit in order to convert the resistance of the variable resistor (110) into a voltage.

5. The apparatus (100) of any of the preceding claims, wherein the transmitter (130) is configured to externally transmit the lung capacity information using a wired or wireless communication method.

6. A wearable spirometer system, comprising
a wearable spirometer apparatus (100) according to any of the preceding claims;
a signal analyzer (3 10) configured to analyze the lung capacity information transmitted from the wearable spirometer apparatus; and
a signal display unit (320) configured to display the analyzed lung capacity information.

7. A method of measuring lung capacity using a wearable spirometer apparatus (100) according to any of claims 1 to 5, comprising:
measuring said resistance of each of said conductive yarns (115);
converting said resistances into said voltages;
converting said voltages in an analog-digital manner and digitizing said converted results;
calculating said changes in cross-sectional capacity of the chest according to said digitized voltages by applying a conversion formula;
adding each calculated change in cross-sectional capacity per height and calculating said lung capacity; and
externally transmitting said calculated lung capacity information.

8. The method of claim 7, further comprising displaying the lung capacity information after calculating the lung capacity.

9. The method of any of claims 7 to 8, further comprising analyzing and displaying the externally transmitted lung capacity information after the calculated lung capacity is externally transmitted.

10. The method of any of claims 7 to 9, wherein the conductive yarns (115) are in the form of a band or clothing.

11. The method of any of claims 7 to 10, wherein the voltages are converted from the resistances by a voltage divider circuit:

12. The method of any of claims 7 to 11, wherein the lung capacity information is externally transmitted using a wired or wireless communication method.

## Patentansprüche

1. Tragbares Spirometergerät (100), umfassend:
Einen variablen Widerstand (110), umfassend eine Mehrzahl von leitfähigen Drähten (115), von denen jeder eine Länge hat, die sich entsprechend einer Veränderung im Brustkorbumfang einer Person beim Atmen verändert und einen Widerstand hat, der sich entsprechend einer Längenänderung des leitfähigen Drahtes verändert, wobei die leitfähigen Drähte in gleichmäßigen Intervallen zueinander beabstandet sind;
einen Widerstand-Spannung-Wandler (122), der dazu ausgelegt ist, den Widerstand jedes der leitfähigen Drähte des variablen Widerstands in eine Spannung umzuwandeln;
einen Analog-Digital-Wandler (124), der dazu ausgelegt ist, jede Spannung in analog-digitaler Weise umzuwandeln und die umgewandelten Ergebnisse zu digitalisieren;
einen Querschnittskapazitätsrechner (126), der dazu ausgelegt ist, Veränderungen der Querschnittskapazität des Brustkorbs einer Person entsprechend den digitalisierten Spannungen durch Anwenden einer Umwandlungsformel zu berechnen;
einen Addierer (128), der dazu ausgelegt ist, jede berechnete Veränderung der Querschnittskapazität pro Höhe aufzuaddieren, und eine Lungenkapazität zu berechnen; und
einen Sender (130), der dazu ausgelegt ist, die berechnete Lungenkapazitätsinformation nach außen zu senden.

2. Gerät (100) nach Anspruch 1, ferner umfassend ein Anzeigemodul (140), das dazu ausgelegt ist, die durch den Addierer berechnete Lungenkapazitätsinformation anzuzeigen.

3. Gerät (100) nach einem der vorhergehenden Ansprüche, wobei der variable Widerstand (110) die Form eines Bands oder Kleidungsstücks hat.

4. Gerät (100) nach einem der vorhergehenden Ansprüche, wobei der Widerstand-Spannung-Wandler (122) eine Spannungsteilerschaltung enthält, um den Widerstand des variablen Widerstands (110) in eine Spannung umzuwandeln.

5. Gerät (100) nach einem der vorhergehenden Ansprüche, wobei der Sender (130) dazu ausgelegt ist, die Lungenkapazitätsinformation unter Verwendung eines drahtgestützten oder drahtlosen Kommunikationsverfahrens nach außen zu senden.

6. Tragbares Spirometersystem, umfassend:
Ein tragbares Spirometergerät (100) nach einem der vorhergehenden Ansprüche;
einen Signalanalysator (310), der dazu ausgelegt ist, die von dem tragbaren Spirometergerät gesendete Lungenkapazitätsinformation zu analysieren; und
eine Signalanzeigeeinheit (320), die dazu ausgelegt ist, die analysierte Lungenkapazitätsinformation anzuzeigen.

7. Verfahren zum Messen einer Lungenkapazität unter Verwendung eines tragbaren Spirometergeräts (100) nach einem der Ansprüche 1-5, umfassend:
Messen des Widerstands jedes der leitfähigen Drähte (115);
Umwandeln der Widerstandswerte in die Spannungen;
Umwandeln der Spannungen in einer analog-digitalen Weise und Digitalisieren der umgewandelten Ergebnisse;
Berechnen der Veränderungen in der Querschnittskapazität des Brustkorbs entsprechend den digitalisierten Spannungen durch Anwenden einer Umwandlungsformel;
Aufaddieren jeder berechneten Veränderung der Querschnittskapazität pro Höhe und Berechnen der Lungenkapazität; und
Senden der berechneten Lungenkapazitätsinformation nach außen.

8. Verfahren nach Anspruch 7, ferner umfassend das Anzeigen der Lungenkapazitätsinformation nach der Berechnung der Lungenkapazität.

9. Verfahren nach einem der Ansprüche 7-8, ferner umfassend das Analysieren und Anzeigen der nach außen gesendeten Lungenkapazitätsinformation, nachdem die berechnete Lungenkapazität nach außen gesendet wurde.

10. Verfahren nach einem der Ansprüche 7-9, wobei die leitfähigen Drähte (115) die Form eines Bands oder Kleidungsstücks haben.

11. Verfahren nach einem der Ansprüche 7-10, wobei die Spannungen aus den Widerstandswerten durch eine Spannungsteilerschaltung umgewandelt werden.

12. Verfahren nach einem der Ansprüche 7-11, wobei die Lungenkapazitätsinformation unter Verwendung eines drahtgestützten oder drahtlosen Kommunikationsverfahrens nach außen gesendet wird.

## Revendications

1. Appareil spiromètre portable (100), comprenant :
un résistor variable (110) incluant une pluralité de fils conducteurs (115), dont chacun a une longueur qui change selon un changement de circonférence de la poitrine d'une personne lorsqu'elle respire, et a une résistance qui change selon un changement de longueur du fil conducteur, dans lequel lesdits fils conducteurs sont espacés à des intervalles uniformes ;
un convertisseur résistance-tension (122) configuré pour convertir la résistance de chacun des fils conducteurs du résistor variable en tension ;
un convertisseur analogique-numérique (124) configuré pour convertir chaque tension de manière analogique-numérique, et numérisant les résultats convertis ;
un calculateur de capacité transversale (126) configuré pour calculer des changements de capacité transversale de la poitrine d'une personne selon les tensions numérisées en appliquant une formule de conversion ;
un additionneur (128) configuré pour additionner chaque changement calculé de capacité transversale par hauteur, et pour calculer une capacité pulmonaire ; et
un transmetteur (130) configuré pour transmettre à l'extérieur l'information de capacité pulmonaire calculée.

2. Appareil (100) selon la revendication 1, comprenant en outre un module d'affichage (140) configuré pour afficher l'information de capacité pulmonaire calculée par l'additionneur.

3. Appareil (100) selon l'une quelconque des revendications précédentes, dans lequel le résistor variable (110) est sous la forme d'une bande ou d'un vêtement.

4. Appareil (100) selon l'une quelconque des revendications précédentes, dans lequel le convertisseur résistance-tension (122) comprend un circuit diviseur de tension afin de convertir la résistance du résistor variable (110) en une tension.

5. Appareil (100) selon l'une quelconque des revendications précédentes, dans lequel le transmetteur (130) est configuré pour transmettre à l'extérieur l'information de capacité pulmonaire en utilisant un procédé de communication filaire ou sans fil.

6. Système de spiromètre portable, comprenant
un appareil spiromètre portable (100) selon l'une quelconque des revendications précédentes ;
un analyseur de signal (310) configuré pour analyser l'information de capacité pulmonaire transmise par l'appareil spiromètre portable ; et
une unité d'affichage de signal (320) configurée pour afficher l'information de capacité pulmonaire analysée.

7. Procédé de mesure de capacité pulmonaire utilisant un appareil spiromètre portable (100) selon l'une quelconque des revendications 1 à 5, comprenant :
la mesure de ladite résistance de chacun desdits fils conducteurs (115) ;
la conversion desdites résistances en lesdites tensions ;
la conversion desdites tensions de manière analogique-numérique et la numérisation desdits résultats convertis ;
le calcul desdits changements de capacité transversale de la poitrine selon lesdites tensions numérisées en appliquant une formule de conversion ;
l'addition de chaque changement calculé de capacité transversale par hauteur et le calcul de ladite capacité pulmonaire ; et
la transmission à l'extérieur de ladite information de capacité pulmonaire calculée.

8. Procédé selon la revendication 7, comprenant en outre l'affichage de l'information de capacité pulmonaire après calcul de la capacité pulmonaire.

9. Procédé selon l'une quelconque des revendications 7 à 8, comprenant en outre l'analyse et l'affichage de l'information de capacité pulmonaire transmise à l'extérieur après la transmission à l'extérieur de la capacité pulmonaire calculée.

10. Procédé selon l'une quelconque des revendications 7 à 9, dans lequel les fils conducteurs (115) sont sous forme d'une bande ou d'un vêtement.

11. Procédé selon l'une quelconque des revendications 7 à 10, dans lequel les tensions sont converties à partir des résistances par un circuit diviseur de tension.

12. Procédé selon l'une quelconque des revendications 7 à 11, dans lequel l'information de capacité pulmonaire est transmise à l'extérieur en utilisant un procédé de communication filaire ou sans fil.
